# NEW EUROPEAN PATENT SPECIFICATION

(11) **EP 1 019 077 B2**
(45) Date of publication and mention of the opposition decision: **22.12.2010**
(45) Mention of the grant of the patent: 28.11.2007
(21) Application number: 98939260.0
(22) Date of filing: 06.08.1998
(51) Int. Cl.: A61K 38/16, C07K 14/46, C07K 14/575

(54) **NOVEL EXENDIN AGONIST COMPOUNDS**
NEUE EXENDINAGONIST VERBINDUNGEN
NOUVEAUX COMPOSES AGONISTES DE L'EXENDINE

(30) Priority: 08.08.1997 US 55404 P
(43) Date of publication of application: 19.07.2000
(60) Divisional application: 06015312.9
(73) Proprietor: AMYLIN PHARMACEUTICALS, INC., San Diego, CA 92121 (US)
(72) Inventor: BEELEY, Nigel, Robert, Arnold, Solana Beach, CA 92131 (US); PRICKETT, Kathryn, S., San Diego, CA 92126 (US)
(74) Representative: HOFFMANN EITLE
(86) International application number: PCT/US1998/016387
(87) International publication number: WO 1999/007404

(56) References cited:
- WO-A-97/46584
- WO-A-98/05351
- WO-A-98/30231
- US-A- 5 424 286
- US-A- 5 424 286
- LAWLER R L ET AL: "Comparison of effects of amylin, glucagon-like peptide-1 (GLP-1) and exendin-4 to inhibit pentagastrin-stimulated gastric acid secretion in rats" GASTROENTEROLOGY, SAUNDERS, PHILADELPHIA, PA,, US, vol. 112, no. 4 SUPPL, 1997, page A194 XP002167251 ISSN: 0016-5085

## Description

### Field of the Invention

The present invention relates to novel compounds which have activity as exendin agonists.

### Exendin

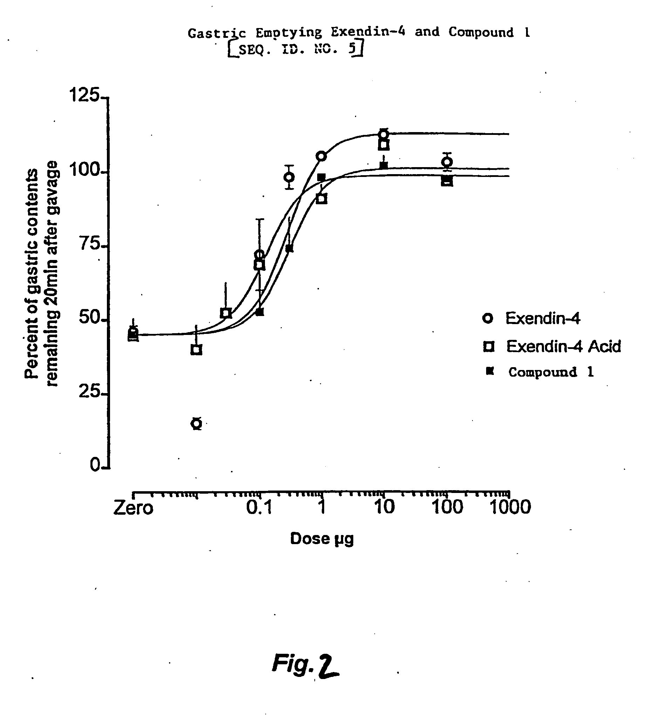

The exendins are peptides that are found in the venom of the Gila-monster, a lizard common in Arizona and Northern Mexico. Exendin-3 [SEQ. ID. NO.1] is present in the venom of Heloderma horridum, and exendin-4 [SEQ. ID. NO. 2] is present in the venom of Heloderma suspectum (Eng, J., et al., J. Biol. Chem., 265:20259-62, 1990; Eng. J., et al., J. Biol. Chem., 267:7402-05, 1992). The amino acid sequence of exendin-3 is shown in Figure 2. The amino acid sequence of exendin-4 is shown in Figure 3. The exendins have some sequence similarity to several members of the glucagon-like peptide family, with the highest homology, 53%, being to GLP-1[7-36]NH₂ [SEQ. ID. NO. 3] (Goke, et al., J. Biol. Chem., 268:19650-55, 1993). GLP-1[7-36]NH₂, also known as proglucagon[78-107] or simply, "GLP-1," has an insulinotropic effect, stimulating insulin secretion from pancreatic β-cells. The amino acid sequence of GLP-1 is shown in Figure 4. GLP-1 also inhibits glucagon secretion from pancreatic α-cells (⌀rsov, et al., Diabetes, 42:658-61, 1993; D'Alessio, et al., J. Clin, Invest., 97:133-38, 1996). GLP-1 is reported to inhibit gastric emptying (Willms B, et al., J Clin Endocrinol Metab 81 (1): 327-32, 1996; Wettergren A, et al., Dig Dis Sci 38 (4): 665-73, 1993), and gastric acid secretion. Schjoldager BT, et al. , Dig Dis Sci 34 (5) : 7-03-8, 1989; O'Halloran DJ, et al., J Endocrinol 126 (1): 169-73, 1990; Wettergren A, et al., Dig Dis Sci 38 (4): 665-73, 1993). GLP-1[7-37], which has an additional glycine residue at its carboxy terminus, also stimulates insulin secretion in humans (⌀rsov, et al., Diabetes, 42:658-61, 1993). A transmembrane G-protein adenylate-cyclase-coupled receptor believed to be responsible for the insulinotropic effect of GLP-1 has been cloned from a β-cell line (Thorens, Proc. Natl. Acad. Sci. USA 89:8641-45 (1992)).

Exendin-4 reportedly acts at GLP-1 receptors on insulin-secreting βTC1 cells, at dispersed acinar cells from guinea pig pancreas, and at parietal cells from stomach; the peptide is also said to stimulate somatostatin release and inhibit gastrin release in isolated stomachs (Goke, et al., J. Biol Chem. 268:19650-55, 1993; Schepp, et al., Eur. J. Pharmacol., 69:183-91, 1994; Eissele, et al., Life Sci., 55:629-34, 1994). Exendin-3 and exendin-4 were reportedly found to stimulate cAMP production in, and amylase release from, pancreatic acinar cells (Malhotra, R., et al., Regulatory Peptides,41:149-56, 1992; Raufman, et al., J. Biol. Chem. 267:21432-37, 1992; Singh, et al., Regul. Pept. 53:47-59, 1994). Based on their insulinotropic activities, the use of exendin-3 and exendin-4 for the treatment of diabetes mellitus and the prevention of hyperglycemia has been proposed (Eng, U.S. Patent No. 5,424,286).

Agents which serve to delay gastric emptying have found a place in medicine as diagnostic aids in gastro-intestinal radiologic examinations. For example, glucagon is a polypeptide hormone which is produced by the α cells of the pancreatic islets of Langerhans. It is a hyperglycemic agent which mobilizes glucose by activating hepatic glycogenolysis. It can to a lesser extent stimulate the secretion of pancreatic insulin. Glucagon is used in the treatment of insulin-induced hypoglycemia, for example, when administration of glucose intravenously is not possible. However, as glucagon reduces the motility of the gastro-intestinal tract it is also used as a diagnostic aid in gastro-intestinal radiological examinations. Glucagon has also been used in several studies to treat various painful gastro-intestinal disorders associated with spasm. Daniel, et al. (Br. Med. J., 3:720, 1974) reported quicker symptomatic relief of acute diverticulitis in patients treated with glucagon compared with those who had been treated with analgesics or antispasmodics. A review by Glauser, et al., (J. Am. Coll. Emergency Physns, 8:228, 1979) described relief of acute esophageal food obstruction following glucagon therapy. In another study glucagon significantly relieved pain and tenderness in 21 patients with biliary tract disease compared with 22 patients treated with placebo (M.J. Stower, et al., Br. J. Surg., 69:591-2, 1982).

Methods for regulating gastrointestinal motility using amylin agonists are described in International Application No. WO95/07098, published March 16, 1995.

Methods for regulating gastrointestinal motility using exendin agonists are described in U.S. 6,858,576.

Certain exendin agonists are described in WO93/25727 and in WO99/25728.

### SUMMARY OF THE INVENTION

According to the present invention, provided are peptide compounds of the formula (I) [SEQ ID NO: 4], wherein said peptide compound exhibits exendin agonist activity as an agent to decrease gastric motility and slow gastric emptying, wherein the compound has an amino acid sequence selected from SEQ ID NOs: 5, 6 and 7.

The present invention further relates to the use of these compounds for the manufacture of a medicament for use in the regulation of gastrointestinal motility associated with a disorder in which a decrease in gastrointestinal motility would be therapeutic. For example regulation of gastro-intestinal motility may comprise reducing gastric motility or slowing gastric emptying.

### BRIEF DESCRIPTION OF THE DRAWINGS

Figure 1 depicts dose dependent effects of exendin-4 in comparison with compound 1 of Figure 1 [SEQ. ID. NO. 5] on plasma glucose levels in db/db mice.
Figure 2 depicts a comparison of effects on gastric emptying of exendin-4, exendin-4 acid and compound 1 of Figure 1 [SEQ. ID. NO.5].

### DETAILED DESCRIPTION OF THE INVENTION

According to the present invention, provided are peptide compounds having an amino acid sequence selected from SEQ ID NOs: 5, 6 and 7. The invention further provides compositions comprising these compounds and a pharmaceutically acceptable carrier.

The present invention also relates to the use of these compounds for the manufacture of a medicament for use in the regulation of gastrointestinal motility associated with a disorder in which a decrease in gastrointestinal motility would be therapeutic. For example regulation of gastro-intestinal motility may comprise reducing gastric motility or slowing gastric emptying.

The compounds referenced above form salts with various inorganic and organic acids and bases. Such salts include salts prepared with organic and inorganic acids, for example, HCl, HBr, H₂SO₄, H₃PO₄, trifluoroacetic acid, acetic acid, formic acid, methanesulfonic acid, toluenesulfonic acid, maleic acid, fumaric acid and camphorsulfonic acid. Salts prepared with bases include ammonium salts, alkali metal salts, e.g., sodium and potassium salts, and alkali earth salts, e.g., calcium and magnesium salts. Acetate, hydrochloride, and trifluoroacetate salts are preferred. The salts may be formed by conventional means, as by reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

### Utility

The compounds described above are useful in view of their pharmacological properties. In particular, the compounds of the invention are exendin agonists, and possess activity as agents to regulate gastric motility and to slow gastric emptying, as evidenced by the ability to reduce post-prandial glucose levels in mammals.

### Preparation of Compounds

The compounds of the present invention may be prepared using standard solid-phase peptide synthesis techniques and preferably an automated or semiautomated peptide synthesizer. Typically, using such techniques, an α-N-carbamoyl protected amino acid and an amino acid attached to the growing peptide chain on a resin are coupled at room temperature in an inert solvent such as dimethylformamide, N-methylpyrrolidinone or methylene chloride in the presence of coupling agents such as dicyclohexylcarbodiimide and 1-hydroxybenzotriazole in the presence of a base such as diisopropylethylamine. The α-N-carbamoyl protecting group is removed from the resulting peptide-resin using a reagent such as trifluoroacetic acid or piperidine, and the coupling reaction repeated with the next desired N-protected amino acid to be added to the peptide chain. Suitable N-protecting groups are well known in the art, with t-butyloxycarbonyl (tBoc) and fluorenylmethoxycarbonyl (Fmoc) being preferred herein.

The solvents, amino acid derivatives and 4-methylbenzhydryl-amine resin used in the peptide synthesizer may be purchased from Applied Biosystems Inc. (Foster City, CA). The following side-chain protected amino acids may be purchased from Applied Biosystems, Inc.: Boc-Arg(Mts), Fmoc-Arg(Pmc), Boc-Thr(Bzl), Fmoc-Thr(t-Bu), Boc-Ser(Bzl), Fmoc-Ser(t-Bu), Boc-Tyr(BrZ), Fmoc-Tyr(t-Bu), Boc-Lys(Cl-Z), Fmoc-Lys(Boc), Boc-Glu(Bzl), Fmoc-Glu(t-Bu), Fmoc-His(Trt), Fmoc-Asn(Trt), and Fmoc-Gln(Trt). Boc-His(BOM) may be purchased from Applied Biosystems, Inc. or Bachem Inc. (Torrance, CA). Anisole, dimethylsulfide, phenol, ethanedithiol, and thioanisole may be obtained from Aldrich Chemical Company (Milwaukee, WI). Air Products and Chemicals (Allentown, PA) supplies HF. Ethyl ether, acetic acid and methanol may be purchased from Fisher Scientific (Pittsburgh, PA).

Solid phase peptide synthesis may be carried out with an automatic peptide synthesizer (Model 430A, Applied Biosystems Inc., Foster City, CA) using the NMP/HOBt (Option 1) system and tBoc or Fmoc chemistry (see, Applied Biosystems User's Manual for the ABI 430A Peptide Synthesizer, Version 1.3B July 1, 1988, section 6, pp. 49-70, Applied Biosystems, Inc., Foster City, CA) with capping. Boc-peptide-resins may be cleaved with HF (-5°C to 0°C, 1 hour). The peptide may be extracted from the resin with alternating water and acetic acid, and the filtrates lyophilized. The Fmoc-peptide resins may be cleaved according to standard methods (Introduction to Cleavage Techniques, Applied Biosystems, Inc., 1990, pp. 6-12). Peptides may be also be assembled using an Advanced Chem Tech Synthesizer (Model MPS 350, Louisville, Kentucky).

Peptides may be purified by RP-HPLC (preparative and analytical) using a Waters Delta Prep 3000 system. A C4, C8 or C18 preparative column (10 *µ*, 2.2 x 25 cm; Vydac, Hesperia, CA) may be used to isolate peptides, and purity may be determined using a C4, C8 or C18 analytical column (5 *µ*, 0.46 x 25 cm; Vydac). Solvents (A=0.1% TFA/water and B=0.1% TFA/CH₃CN) may be delivered to the analytical column at a flowrate of 1.0 ml/min and to the preparative column at 15 ml/min. Amino acid analyses may be performed on the Waters Pico Tag system and processed using the Maxima program. Peptides may be hydrolyzed by vapor-phase acid hydrolysis (115°C, 20-24 h). Hydrolysates may be derivatized and analyzed by standard methods (Cohen, et al., The Pico Tag Method- A Manual of Advanced Techniques for Amino Acid Analysis, pp. 11-52, Millipore Corporation, Milford, MA (1989)). Fast atom bombardment analysis may be carried out by M-Scan, Incorporated (West Chester, PA). Mass calibration may be performed using cesium iodide or cesium iodide/glycerol. Plasma desorption ionization analysis using time of flight detection may be carried out on an Applied Biosystems Bio-Ion 20 mass spectrometer. Electrospray mass spectroscopy may be carried and on a VG-Trio machine.

Peptide compounds useful in the invention may also be prepared using recombinant DNA techniques, using methods now known in the art. See, e.g., Sambrook et al., Molecular Cloning: A Laboratory Manual, 2d Ed., Cold Spring Harbor

The compounds referenced above may form salts with various inorganic and organic acids and bases. Such salts include salts prepared with organic and inorganic acids, for example, HCl, HBr, H₂SO₄, H₃PO₄, trifluoroacetic acid, acetic acid, formic acid, methanesulfonic acid, toluenesulfonic acid, maleic acid, fumaric acid and camphorsulfonic acid. Salts prepared with bases include ammonium salts, alkali metal salts, e.g., sodium and potassium salts, and alkali earth salts, e.g., calcium and magnesium salts. Acetate, hydrochloride, and trifluoroacetate salts are preferred. The salts may be formed by conventional means, as by reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

### Formulation and Administration

Compounds of the invention are useful in view of their exendin-like effects, and may conveniently be provided in the form of formulations suitable for parenteral (including intravenous, intramuscular and subcutaneous) or nasal or oral administration. In some cases, it will be convenient to provide an exendin or exendin agonist and another anti-gastric-emptying agent, such as glucagon, an amylin, or an amylin agonist, in a single composition or solution for administration together. In other cases, it may be more advantageous to administer another anti-emptying agent separately from said exendin or exendin agonist. In yet other cases, it may be beneficial to provide an exendin or an exendin agonist either co-formulated or separately with other glucose lowering agents such as insulin. A suitable administration format may best be determined by a medical practitioner for each patient individually. Suitable pharmaceutically acceptable carriers and their formulation are described in standard formulation treatises, e.g., Remington's Pharmaceutical Sciences by E.W. Martin. See also Wang, Y.J. and Hanson, M.A. "Parenteral Formulations of Proteins and Peptides: Stability and Stabilizers," Journal of Parenteral Science and Technology, Technical Report No. 10, Supp. 42:25 (1988).

Compounds useful in the invention can be provided as parenteral compositions for injection or infusion. They can, for example, be suspended in an inert oil, suitably a vegetable oil such as sesame, peanut, olive oil, or other acceptable carrier. Preferably, they are suspended in an aqueous carrier, for example, in an isotonic buffer solution at a pH of about 5.6 to 7.4. These compositions may be sterilized by conventional sterilization techniques, or may be sterile filtered. The compositions may contain pharmaceutically acceptable auxiliary substances as required to approximate physiological conditions, such as pH buffering agents. Useful buffers include for example, sodium acetate/acetic acid buffers. A form of repository or "depot" slow release preparation may be used so that therapeutically effective amounts of the preparation are delivered into the bloodstream over many hours or days following transdermal injection or delivery.

The desired isotonicity may be accomplished using sodium chloride or other pharmaceutically acceptable agents such as dextrose, boric acid, sodium tartrate, propylene glycol, polyols (such as mannitol and sorbitol), or other inorganic or organic solutes. Sodium chloride is preferred particularly for buffers containing sodium ions.

The claimed compounds can also be formulated as pharmaceutically acceptable salts (e.g., acid addition salts) and/or complexes thereof. Pharmaceutically acceptable salts are non-toxic salts at the concentration at which they are administered. The preparation of such salts can facilitate the pharmacological use by altering the physical-chemical characteristics of the composition without preventing the composition from exerting its physiological effect. Examples of useful alterations in physical properties include lowering the melting point to facilitate transmucosal administration and increasing the solubility to facilitate the administration of higher concentrations of the drug.

Pharmaceutically acceptable salts include acid addition salts such as those containing sulfate, hydrochloride, phosphate, sulfamate, acetate, citrate, lactate, tartrate, methanesulfonate, ethanesulfonate, benzenesulfonate, *p*-toluenesulfonate, cyclohexylsulfamate and quinate. Pharmaceutically acceptable salts can be obtained from acids such as hydrochloric acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, and quinic acid. Such salts may be prepared by, for example, reacting the free acid or base forms of the product with one or more equivalents of the appropriate base or acid in a solvent or medium in which the salt is insoluble, or in a solvent such as water which is then removed in vacuo or by freeze-drying or by exchanging the ions of an existing salt for another ion on a suitable ion exchange resin.

Carriers or excipients can also be used to facilitate administration of the compound. Examples of carriers and excipients include calcium carbonate, calcium phosphate, various sugars such as lactose, glucose, or sucrose, or types of starch, cellulose derivatives, gelatin, vegetable oils, polyethylene glycols and physiologically compatible solvents. The compositions or pharmaceutical composition can be administered by different routes including intravenously, intraperitoneal, subcutaneous, and intramuscular, orally, topically, or transmucosally.

If desired, solutions of the above compositions may be thickened with a thickening agent such as methyl cellulose. They may be prepared in emulsified form, either water in oil or oil in water. Any of a wide variety of pharmaceutically acceptable emulsifying agents may be employed including, for example, acacia powder, a non-ionic surfactant (such as a Tween), or an ionic surfactant (such as alkali polyether alcohol sulfates or sulfonates, e.g., a Triton).

Compositions useful in the invention are prepared by mixing the ingredients following generally accepted procedures. For example, the selected components may be simply mixed in a blender or other standard device to produce a concentrated mixture which may then be adjusted to the final concentration and viscosity by the addition of water or thickening agent and possibly a buffer to control pH or an additional solute to control tonicity.

For use by the physician, the compounds will be provided in dosage unit form containing an amount of an exendin agonist, with or without another anti-emptying agent. Therapeutically effective amounts of an exendin agonist for use in the control of gastric emptying and in conditions in which gastric emptying is beneficially slowed or regulated are those that decrease post-prandial blood glucose levels, preferably to no more than about 8 or 9 mM or such that blood glucose levels are reduced as desired. In diabetic or glucose intolerant individuals, plasma glucose levels are higher than in normal individuals. In such individuals, beneficial reduction or "smoothing" of post-prandial blood glucose levels, may be obtained. As will be recognized by those in the field, an effective amount of therapeutic agent will vary with many factors including the age and weight of the patient, the patient's physical condition, the blood sugar level or level of inhibition of gastric emptying to be obtained, and other factors.

Such pharmaceutical compositions are useful in causing gastric hypomotility in a subject and may be used as well in other disorders where gastric motility is beneficially reduced.

The effective daily anti-emptying dose of the compounds will typically be in the range of 0.01 or 0.03 to about 5 mg/day, preferably about 0.01 or 0.5 to 2 mg/day and more preferably about 0.01 or 0.1 to 1 mg/day, for a 70 kg patient, administered in a single or divided doses. The exact dose to be administered is determined by the attending clinician and is dependent upon where the particular compound lies within the above quoted range, as well as upon the age, weight and condition of the individual. Administration should begin at the first sign of symptoms or shortly after diagnosis of diabetes mellitus. Administration may be by injection, preferably subcutaneous or intramuscular. Orally active compounds may be taken orally, however dosages should be increased 5-10 fold.

Generally, in treating or preventing elevated, inappropriate, or undesired post-prandial blood glucose levels, the compounds of this invention may be administered to patients in need of such treatment in a dosage ranges similar to those given above, however, the compounds are administered more frequently, for example, one, two, or three times a day.

The optimal formulation and mode of administration of compounds of the present application to a patient depend on factors known in the art such as the particular disease or disorder, the desired effect, and the type of patient. While the compounds will typically be used to treat human patients, they may also be used to treat similar or identical diseases in other vertebrates such as other primates, farm animals such as swine, cattle and poultry, and sports animals and pets such as horses, dogs and cats.

To assist in understanding the present invention the following Examples are included which describe the results of a series of experiments. hereinafter claimed.

### EXAMPLE 1

### Preparation of amidated peptide having SEQ ID. NO. [5]

The above-identified peptide was assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.). In general, single-coupling cycles were used throughout the synthesis and Fast Moc (HBTU activation) chemistry was employed. However, at some positions coupling was less efficient than expected and double couplings were required. In particular, residues Asp₉, Thr₇ and Phe₆ all required double coupling. Deprotection (Fmoc group removal)of the growing peptide chain using piperidine was not always efficient. Double deprotection was required at positions Arg₂₀, Val₁₉ and Leu₁₄. Final deprotection of the completed peptide resin was achieved using a mixture of triethylsilane (0.2 mL), ethanedithiol (0.2 mL), anisole (0.2 mL), water (0.2 mL) and trifluoroacetic acid (15 mL) according to standard methods (Introduction to Cleavage Techniques, Applied Biosystems, Inc.) The peptide was precipitated in ether/water (50 mL) and centrifuged. The precipitate was reconstituted in glacial acetic acid and lyophilized. The lyophilized peptide was dissolved in water). Crude purity was about 55%.

Used in purification steps and analysis were Solvent A (0.1% TFA in water) and Solvent B (0.1% TFA in ACN).

The solution containing peptide was applied to a preparative C-18 column and purified (10% to 40% Solvent B in Solvent A over 40 minutes). Purity of fractions was determined isocratically using a C-18 analytical column. Pure fractions were pooled furnishing the above-identified peptide. Analytical RP-HPLC (gradient 30% to 60% Solvent B in Solvent A over 30 minutes) of the lyophilized peptide gave product peptide having an observed retention time of 14.5 minutes. Electrospray Mass Spectrometry (M): calculated 4131.7; found 4129.3.

### EXAMPLE 2

### Preparation of Peptide having SEQ. ID. NO. [6]

The above-identified peptide was assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Example 1. Used in analysis were Solvent A (0.1% TFA in water) and Solvent B (0.1% TFA in ACN). Analytical RP-HPLC (gradient 25% to 75% Solvent B in Solvent A over 30 minutes) of the lyophilized peptide gave product peptide having an observed retention time of 21.5 minutes. Electrospray Mass Spectrometry (M): calculated 4168.6; found 4171.2.

### EXAMPLE 3

### Preparation of Peptide having SEQ. ID. NO. [7]

The above-identified peptide was assembled on 4-(2'-4'-dimethoxyphenyl)-Fmoc aminomethyl phenoxy acetamide norleucine MBHA resin (Novabiochem, 0.55 mmole/g) using Fmoc-protected amino acids (Applied Biosystems, Inc.), cleaved from the resin, deprotected and purified in a similar way to Example 1. Used in analysis were Solvent A (0.1% TFA in water) and Solvent B (0.1% TFA in ACN). Analytical RP-HPLC (gradient 30% to 60% Solvent B in Solvent A over 30 minutes) of the lyophilized peptide gave product peptide having an observed retention time of 17.9 minutes. Electrospray Mass Spectrometry (M): calculated 4147.6; found 4150.2.

### EXAMPLE 12

### Preparation of C-terminal carboxylic acid Peptidescorresponding to the above C-terminal amide sequences.

The above peptides of Examples 1 to 3 are assembled on the so called Wang resin (p-alkoxybenzylalacohol resin (Bachem, 0.54 mmole/g)) using Fmoc-protected amino acids (Applied Biosystems, Inc.), cleaved from the resin, deprotected and purified in a similar way to Example 1. Used in analysis are Solvent A (0.1% TFA in water) and Solvent B (0.1% TFA in ACN). Analytical RP-HPLC (gradient 30% to 60% Solvent B in Solvent A over 30 minutes) of the lyophilized peptide is then carried out to determine the retention time of the product peptide. Electrospray Mass Spectrometry provides an experimentally determined (M).

### EXAMPLES A TO D

### Reagents Used

GLP-1 was purchased from Bachem (Torrance, CA), all other peptides were prepared in house using synthesis methods such as those described therein. All chemicals were of the highest commercial grade. The cAMP SPA immunoassay was purchased from Amersham. The radioligands were purchased from New England Nuclear (Boston, MA). RINm5f cells (American Type Tissue Collection, Rockville, MD) were grown in DME/F12 medium containing 10% fetal bovine serum and 2mM L-glutamine. Cells were grown at 37°C and 5% CO₂/95% humidified air and medium was replaced every 2 to 3 days. Cells were grown to confluence then harvested and homogenized using on a Polytron homogenizer. Cell homogenates were stored frozen at -70°C until used.

### Example A

### GLP-1 Receptor Binding Studies

Receptor binding was assessed by measuring displacement of [¹²⁵I] human GLP-1 (7-36) or [¹²⁵I] Exendin (9-39) from RINm5f membranes. Assay buffer contained 5 µg/ml bestatin 1 µg.ml phosphoramidon, 1 mg/ml bovine serum albumin (fraction V), 1 mg/ml bacitracin, and 1 mM MgCl₂ in 20 mM HEPES, pH 7.4. To measure binding, 30 µg membrane protein (Bradford protein assay) was resuspended in 200 µl assay buffer and incubated with 60 pM [¹²⁵I] human GLP-1 or Exendin (9-39) and unlabeled peptides for 120 minutes as 23°C in 96 well plates (Nagle Nunc, Rochester, NY). Incubations were terminated by rapid filtration with cold phosphatebuffered saline, pH 7.4, through polyethyleneimine-treated GF/B glass fiber filters (Wallac Inc., Gaithersburg, MD) using a Tomtec Mach II plate harvester (Wallac Inc., Gaithersburg, MD). Filters were dried, combined with scintillant, and radioactivity determined in a Betaplate liquid scintillant counter (Wallac Inc.).

Peptide samples were run in the assay as duplicate points at 6 dilutions over a concentration range of 10⁻⁶M to 10⁻¹²M to generate response curves. The biological activity of a sample is expressed as an IC₅₀ value, calculated from the raw data using an iterative curve-fitting program using a 4-parameter logistic equation (Prism, GraphPAD Software).

### Example B

### Cyclase Activation Study

Assay buffer contained 10 *µ*M GTP, 0.75 mM ATP, 2.5 mM MgCl₂, 0.5mM phosphocreatine, 12,5 U/ml creatine kinase, 0.4 mg/ml aprotinin, 1 *µ*M IBMX in 50 mM HEPES, pH 7.4. Membranes and peptides were combined in 100 ml of assay buffer in 96 well filter-bottom plates (Millipore Corp., Bedford, MA). After 20 minutes incubation at 37°C, the assay was terminated by transfer of supernatant by filtration into a fresh 96 well plate using a Millipore vacuum manifold. Supernatant cAMP contents were quantitated by SPA immunoassay.

Peptide samples were run in the assay as triplicate points at 7 dilutions over a concentration range of 10⁻⁶M to 10⁻¹²M to generate response curves. The biological activity of a particular sample was expressed as an EC₅₀ value, calculated as described above. Results are tabulated in Table I.

**TABLE I**

| Activity in the RINm5f cyclase assay | |
|---|---|
| | EC₅₀ |
| Exendin-4 [SEQ. ID. NO. 2] | 0.23 |
| Compound 1 [SEQ. ID. NO. 5] | 0.17 |
| Compound 2 [SEQ. ID. NO. 6] | 0.23 |
| Compound 3 [SEQ. ID. NO. 7] | 0.42 |

### Example C

### Determination of Blood Glucose Levels in db/db Mice - 1 Hour Protocol

C57BL/6J-m=/=Lepr^{db} mice, at least 3 months of age were utilized for the study. The mice were obtained from The Jackson Laboratory and allowed to acclimate for at least one week in the vivarium. Mice were housed in groups of ten at 22° ±1°C with a 12:12 light:dark cycle, with lights on at 6 a.m.

All animals were deprived of food for 2 hours before taking baseline blood samples. Approximately 100 *µ*l of blood was drawn from each mouse via eye puncture, after a light anesthesia with metophane. After collecting baseline blood samples, to measure plasma glucose concentrations, all animals receive subcutaneous injections of either vehicle, exendin-4 or test compound in concentrations indicated. Blood samples were drawn again, using the same procedure, after exactly one hour from the injections, and plasma glucose concentrations were measured.

For each animal, the % change in plasma value, from baseline value, was calculated and a dose dependent relationship was evaluated using Graphpad Prizm^{™} software.

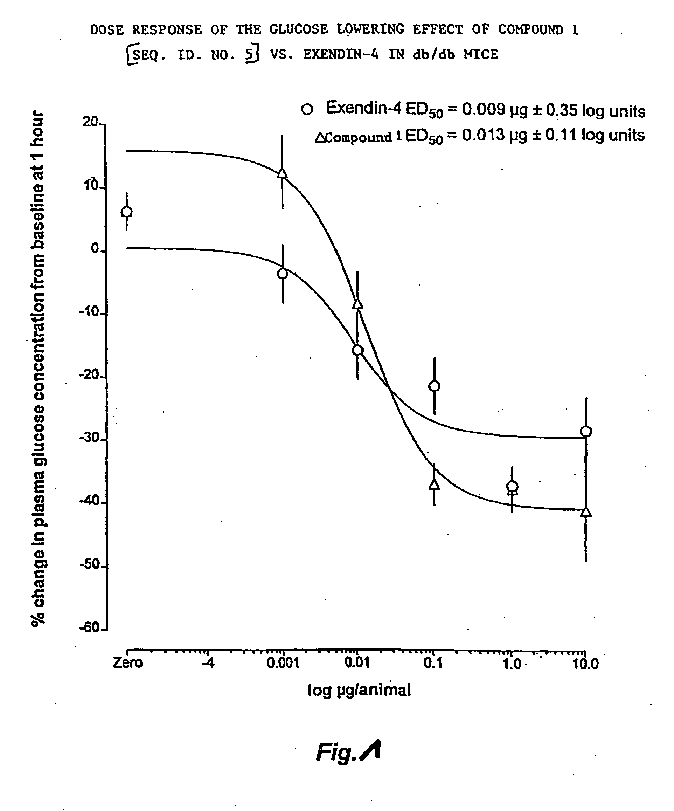

Figure 1 depicts the effects of varying doses of exendin-4 and Compound 1 [SEQ. ID. NO. 5] on plasma glucose levels.

### Example D

The following study was carried out to examine the effects of exendin-4, exendin-4 acid and an exendin agonist (Compound 1 [SEQ. ID. NO. 5])on gastric emptying in rats. This experiment followed a modification of the method of Scarpignato, et al., Arch. Int. Pharmacodyn. Ther. 246:286-94 (1980) .

Male Harlan Sprague Dawley (HSD) rats were used. All animals were housed at 22.7±0.8 C in a 12:12 hour light:dark cycle (experiments being performed during the light cycle) and were fed and watered *ad libitum* (Diet LM-485, Teklad, Madison, WI). Exendin-4 and exendin-4 acid were synthesized according to standard peptide synthesis methods. The preparation of Compound 1 [SEQ. ID. NO. 5] is described in Example 1.

The determination of gastric emptying by the method described below was performed after a fast of ~20 hours to ensure that the stomach contained no chyme that would interfere with spectrophotometric absorbance measurements.

Conscious rats received by gavage, 1.5ml of an acaloric gel containing 1.5% methyl cellulose (M-0262, Sigma Chemical Co, St Louis, MO) and 0.05% phenol red indicator. Twenty minutes after gavage, rats were anesthetized using 5% halothane, the stomach exposed and clamped at the pyloric and lower esophageal sphincters using artery forceps, removed and opened into an alkaline solution which was made up to a fixed volume. Stomach content was derived from the intensity of the phenol red in the alkaline solution, measured by absorbance at a wavelength of 560 nm. In separate experiments on 7 rats, the stomach and small intestine were both excised and opened into an alkaline solution. The quantity of phenol red that could be recovered from the upper gastrointestinal tract within 20 minutes of gavage was 89±4%; dye which appeared to bind irrecoverably to the gut luminal surface may have accounted for the balance. To account for a maximal dye recovery of less than 100%, percent of stomach contents remaining after 20 min were expressed as a fraction of the gastric contents recovered from control rats sacrificed immediately after gavage in the same experiment. Percent gastric contents remaining = (absorbance at 20 min)/(absorbance at 0 mm) x 100.

In baseline studies, with no drug treatment, gastric emptying over 20 min was determined. In dose-response studies, rats were treated with 0.01, 0.1, 0.3, 1, 10 and 100 µg of exendin-4, 0.01, 0.03, 0.1, 1, 10 and 100 µg exendin-4 acid, and 0.1, 0.3, 1, 10 and 100 µg of Compound 1 [SEQ. ID. NO. 5] .

The results are shown in Figure 2. The results, shown in Figure 2 and Table II, show that the exendin agonists, exendin-4 acid and compound 1 are potent inhibitors of gastric emptying. The EC₅₀ of exendin-4 was 0.27 µg. The EC₅₀s of exendin-4 acid and Compound 1 were comparable (0.12 µg and 0.29 µg, respectively).

**TABLE II**

| Compound | EC₅₀ (µg) |
|---|---|
| exendin-4 | 0.27 |
| exendin-4 acid | 0.12 |
| Conpound 1 | 0.29 |

## Claims

1. A peptide compound of the formula (I) [SEQ ID NO: 4], wherein said peptide compound exhibits exendin agonist activity as an agent to decrease gastrointestinal motility and slow gastric emptying, wherein the compound has an amino acid sequence selected from SEQ ID Nos: 5, 6 and 7.

2. A composition comprising a compound of claim 1 in a pharmaceutically acceptable carrier.

3. Use of a compound of claim 1 in the manufacture of a medicament for use in the regulation of gastrointestinal motility associated with a disorder in which a decrease in gastrointestinal motility would be therapeutic.

4. Use according to claim 3, wherein said regulation of gastrointestinal motility comprises slowing gastric emptying.

## Patentansprüche

1. Peptidverbindung der Formel (I) (SEQ ID NO: 4), wobei die Peptidverbindung Exendin-Agonistaktivität aufweist, als Wirkstoff zur Herabsetzung der gastrointestinalen Motilität und zur Verlangsamung der Magenentleerung, wobei die Verbindung eine Aminosäuresequenz, ausgewählt aus SEQ ID NOS: 5, 6 und 7, hat.

2. Zusammensetzung, umfassend eine Verbindung gemäss Anspruch 1, in einem pharmazeutisch akzeptablen Träger.

3. Verwendung einer Verbindung gemäss Anspruch 1 zur Herstellung eines Medikaments zur Verwendung bei der Regulation der gastrointestinalen Motilität im Zusammenhang mit einer Funktionsstörung, bei der die Herabsetzung der gastrointestinalen Motilität therapeutisch wäre.

4. Verwendung gemäss Anspruch 3, wobei die Regulation der gastrointestinalen Motilität eine Verlangsamung der Magenentleerung umfasst.

## Revendications

1. Composé peptidique de formule (1) (SEQ ID n° 4), lequel composé peptidique présentant une activité d'agoniste de l'exendine comme agent pour réguler la motilité gastrique et ralentir l'évacuation gastrique, dans lequel le composé a une séquence d'acides aminés choisie parmi les SEQ ID n° 5, 6 et 7.

2. Composition comprenant un composé de la revendication 1 dans un véhicule pharmaceutiquement acceptable.

3. Utilisation d'un composé de la revendication 1 dans la fabrication d'un médicament à utiliser dans la régulation de la motilité gastro-intestinale associée à un trouble dans lequel une augmentation ou une diminution de la motilité gastro-intestinale serait thérapeutique.

4. Utilisation selon la revendication 3, dans laquelle ladite régulation de la motilité gastro-intestinale comprend le ralentissement de l'évacuation gastrique.


### SEQUENCE LISTING

<110> Amylin Pharmaceuticals, Inc.
<120> NOVEL EXENDIN AGONIST COMPOUNDS
<130> P17422
<140> EP 19980939260
   <141 > 1998-08-06
<150> US 60/055,404
   <151> 1997-08-08
<160> 40
<170> PatentIn version 3.2
<210> 1
   <211> 39
   <212> PRT
   <213> Heloderma horridum
<220>
   <221> MISC FEATURE
   <223> c-term amidation
<400> 1
   His Ser Asp Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 2
   <211> 39
   <212> PRT
   <213> Heloderma suspectum
<220>
   <221> MISC_FEATURE
   <223> c-term amidation
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 3
   <211> 30
   <212> PRT
   <213> Homo sapiens
<220>
   <221> MISC_FEATURE
   <223> c-term amidation
<400> 3
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 4
   <211> 39
   <212> PRT
   <213> Artificial
<220>
   <223> Artificial Sequence
<220>
   <221> MISC_FEATURE
   <223> Description of Artificial Sequence: Exendin agonist formula peptide
<220>
   <221> MISC_FEATURE
   <223> c-term may be amidated
<220>
   <221> MISC_FEATURE
   <223> See specification as filed for detailed description of substitutions and preferred embodiments
<220>
   <221> MISC_FEATURE
   <222> (1)..(1)
   <223> His, Arg or Tyr
<220>
   <221> MISC_FEATURE
   <222> (2)..(2)
   <223> Ser, Gly, Ala or Thr
<220>
   <221> MISC_FEATURE
   <222> (3)..(3)
   <223> Asp or Glu
<220>
   <221> MISC_FEATURE
   <222> (6)..(6)
   <223> Phe, Tyr or naphthylalanine
<220>
   <221> MISC_FEATURE
   <222> (7)..(7)
   <223> Thr or Ser
<220>
   <221> MISC_FEATURE
   <222> (8)..(8)
   <223> Ser or Thr
<220>
   <221> MISC_FEATURE
   <222> (9)..(9)
   <223> Asp or Glu
<220>
   <221> MISC_FEATURE
   <222> (10)..(10)
   <223> Leu, Ile, Val, pentylglycine or Met
<220>
   <221> MISC_FEATURE
   <222> (14)..(14)
   <223> Leu, Ile, pentylglycine, Val or Met
<220>
   <221> MISC_FEATURE
   <222> (22)..(22)
   <223> Phe, Tyr or naphthylalanine
<220>
   <221> MISC_FEATURE
   <222> (23)..(23)
   <223> Ile, Val, Leu, pentylglycine, tert-butylglycine or Met
<220>
   <221> MISC_FEATURE
   <222> (24)..(24)
   <223> Glu or Asp
<220>
   <221> MISC_FEATURE
   <222> (25)..(25)
   <223> Trp, Phe, Tyr or naphthylalanine
<220>
   <221> MISC_FEATURE
   <222> (31)..(31)
   <223> Pro, homoproline, 3Hyp, 4Hyp, thioproline, N-alkylglycine, N-alkylpentylglycine or N-alkylalanine
<220>
   <221> MISC_FEATURE
   <222> (36)..(38)
   <223> Pro, homoproline, 3Hyp, 4Hyp, thioproline, N-alkylglycine, N-alkylpentylglycine or N-alkylalanine
<220>
   <221> MISC_FEATURE
   <222> (39)..(39)
   <223> Ser, Thr or Tyr
<400> 4
   Xaa Xaa Xaa Gly Thr Xaa Xaa Xaa Xaa Xaa Ser Lys Gln Xaa Glu Glu
   1 5 10 15
   Glu Ala Val Arg Leu Xaa Xaa Xaa Xaa Leu Lys Asn Gly Gly Xaa Ser
   20 25 30
   Ser Gly Ala Xaa Xaa Xaa Xaa
   35
<210> 5
   <211> 39
   <212> PRT
   <213> Artificial
<220>
   <223> Artificial Sequence
<220>
   <221> MISC_FEATURE
   <223> c-term amidation
<220>
   <221> MISC_FEATURE
   <223> Description of Artificial Sequence: Exendin agonist peptide
<400> 5
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 6
   <211> 39
   <212> PRT
   <213> Artificial
<220>
   <223> Artificial Sequence
<220>
   <221> MISC_FEATURE
   <223> c-term amidation
<220>
   <221> MISC_FEATURE
   <223> Description of Artificial Sequence: Exendin agonist peptide
<400> 6
   His Gly Glu Gly Thr Phe Thr Ser Asp Leu Ser Lys Gln Leu Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 7
   <211> 39
   <212> PRT
   <213> Artificial
<220>
   <223> Artificial Sequence
<220>
   <221> MISC_FEATURE
   <223> c-term amidation
<220>
   <221> MISC_FEATURE
   <223> Description of Artificial Sequence: Exendin agonist peptide
<400> 7
   His Gly Glu Gly Thr Phe Thr Ser Asp Leu Ser Lys Gln Met Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 8
   <211> 39
   <212> PRT
   <213> Artificial
<220>
   <223> Artificial Sequence
<220>
   <221> MISC_FEATURE
   <223> c-term amidation
<220>
   <221> MISC_FEATURE
   <223> Description of Artificial Sequence: Exendin agonist peptide
<400> 8
   Tyr Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 9
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 9
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Tyr
   35
<210> 10
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 10
   His Gly Asp Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
<210> 11
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (6)..(6)
   <223> naphthylalanine
<400> 11
   His Gly Glu Gly Thr Xaa Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 12
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 12
   His Gly Glu Gly Thr Phe Ser Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 13
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221 MISC_EATURE
   <223> c-term amidation
<400> 13
   His Gly Glu Gly Thr Phe Ser Thr Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 14
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 14
   His Gly Glu Gly Thr Phe Thr Thr Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 15
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 15
   His Gly Glu Gly Thr Phe Thr Ser Glu Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 16
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (10)..(10)
   <223> pentylglycine
<400> 16
   His Gly Glu Gly Thr Phe Thr Ser Asp Xaa Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 17
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC FEATURE
   <223> c-term amidation
<220>
   <221> MISC_FEATURE
   <222> (10)..(10)
   <223> pentylglycine
<400> 17
   His Gly Glu Gly Thr Phe Thr Ser Asp Xaa Ser Lys Gln Leu Glu Glu
   1               5                   10                  15
   Glu Ala Val Arg Leu Phe Ile Glu Phe Leu Lys Asn Gly Gly Pro Ser
               20                  25                  30
   Ser Gly Ala Pro Pro Pro Ser
           35
<210> 18
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (14)..(14)
   <223> pentylglycine
<400> 18
   His Gly Glu Gly Thr Phe Thr Ser Asp Leu Ser Lys Gln Xaa Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 19
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (14)..(14)
   <223> pentylglycine
<400> 19
   His Gly Glu Gly Thr Phe Thr Ser Asp Leu Ser Lys Gln Xaa Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 20
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (22)..(22)
   <223> naphthylalanine
<400> 20
   His Gly Glu Gly Thr Phe Thr Ser Asp Leu Ser Lys Gln Met Glu Glu
   1               5                   10                  15
   Glu Ala Val Arg Leu Xaa Ile Glu Trp Leu Lys Asn Gly Gly Pro Ser
               20                  25                  30
   Ser Gly Ala Pro Pro Pro Ser
           35
<210> 21
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 21
   His Gly Glu Gly Thr Phe Thr Ser Asp Leu Ser Lys Gln Met Glu Glu
   1 5 10 15
   Glu Ala Val Arg Leu Phe Val Glu Trp Leu Lys Asn Gly Gly Pro Ser
   20 25 30
   Ser Gly Ala Pro Pro Pro Ser
   35
<210> 22
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 22
   His Gly Glu Gly Thr Phe Thr Ser Asp Leu Ser Lys Gln Leu Glu Glu
   1 5 10 15
   Glu Ala Val Arg Leu Phe Val Glu Phe Leu Lys Asn Gly Gly Pro Ser
   20 25 30
   Ser Gly Ala Pro Pro Pro Ser
   35
<210> 23
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (23)..(23)
   <223> tert-butylglycine
<400> 23
   His Gly Glu Gly Thr Phe Thr Ser Asp Leu Ser Lys Gln Met Glu Glu
   1 5 10 15
   Glu Ala Val Arg Leu Phe Xaa Glu Trp Leu Lys Asn Gly Gly Pro Ser
   20 25 30
   Ser Gly Ala Pro Pro Pro Ser
   35
<210> 24
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (23)..(23)
   <223> tert-butylglycine
<400> 24
   His Gly Glu Gly Thr Phe Thr Ser Asp Leu Ser Lys Gln Leu Glu Glu
   1 5 10 15
   Glu Ala Val Arg Leu Phe Xaa Glu Phe Leu Lys Asn Gly Gly Pro Ser
   20 25 30
   Ser Gly Ala Pro Pro Pro Ser
   35
<210> 25
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 25
   His Gly Glu Gly Thr Phe Thr Ser Asp Leu Ser Lys Gln Met Glu Glu
   1               5                   10                  15
   Glu Ala Val Arg Leu Phe Ile Asp Trp Leu Lys Asn Gly Gly Pro Ser
               20                  25                  30
   Ser Gly Ala Pro Pro Pro Ser
           35
<210> 26
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<400> 26
   His Gly Glu Gly Thr Phe Thr Ser Asp Leu Ser Lys Gln Met Glu Glu
   1               5                   10                  15
   Glu Ala Val Arg Leu Phe Ile Glu Phe Leu Lys Asn Gly Gly Pro Ser
               20                  25                  30
   Ser Gly Ala Pro Pro Pro Ser
           35
<210> 27
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (31)..(31)
   <223> thioproline
<220>
   <221> MISC_FEATURE
   <222> (36).. (38)
   <223> thioproline
<400> 27
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Xaa Ser
               20                  25                  30
   Ser Gly Ala Xaa Xaa Xaa Ser
           35
<210> 28
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (36)..(38)
   <223> thioproline
<400> 28
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Xaa Xaa Xaa Ser
   35
<210> 29
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (31)..(31)
   <223> homoproline
<220>
   <221> MISC_FEATURE
   <222> (36).. (38)
   <223> homoproline
<400> 29
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Xaa Ser
   20 25 30
   Ser Gly Ala Xaa Xaa Xaa Ser
   35
<210> 30
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (36)..(38)
   <223> homoproline
<400> 30
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Xaa Xaa Xaa Ser
   35
<210> 31
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (31)..(31)
   <223> thioproline
<220>
   <221> MISC_FEATURE
   <222> (36)..(38)
   <223> thioproline
<400> 31
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn Gly Gly Xaa Ser
               20                  25                  30
   Ser Gly Ala Xaa Xaa Xaa Ser
           35
<210> 32
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (31)..(31)
   <223> homoproline
<220>
   <221> MISC_FEATURE
   <222> (36)..(38)
   <223> homoproline
<400> 32
   His Gly Glu Gly Thr Phe Thr Ser Asp Leu Ser Lys Gln Leu Glu Glu
   1               5                   10                  15
   Glu Ala Val Arg Leu Phe Ile Glu Phe Leu Lys Asn Gly Gly Xaa Ser
               20                  25                  30
   Ser Gly Ala Xaa Xaa Xaa Ser
           35
<210> 33
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (31)..(31)
   <223> N-methylalanine
<220>
   <221> MISC_FEATURE
   <222> (36)..(38)
   <223> N-methylalanine
<400> 33
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Xaa Ser
               20                  25                  30
   Ser Gly Ala Xaa Xaa Xaa Ser
           35
<210> 34
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC FEATURE
   <223> c-term amidation
<220>
   <221> MISC_FEATURE
   <222> (36)..(38)
   <223> N-methylalanine
<400> 34
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Xaa Xaa Xaa Ser
           35
<210> 35
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (31)..(31)
   <223> N-methylalanine
<220>
   <221> MISC_FEATURE
   <222> (36)..(38)
   <223> N-methylalanine
<400> 35
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Xaa Ser
   20 25 30
   Ser Gly Ala Xaa Xaa Xaa Ser
   35
<210> 36
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (1)..(1)
   <223> 4-imidazopropionyl
<220>
   <221> MISC_FEATURE
   <222> (27)..(27)
   <223> Lys-NH(epsilon) octanoyl
<400> 36
   Xaa Gly Glu Gly Thr Phe Thr Ser Asp Leu Ser Lys Gln Met Glu Glu
   1 5 10 15
   Glu Ala Val Arg Leu Phe Ile Glu Trp Leu Xaa Asn Gly Gly Pro Ser
   20 25 30
   Ser Gly Ala Pro Pro Pro Ser
   35
<210> 37
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (1)..(1)
   <223> 4-imidazopropionyl
<220>
   <221> MISC_FEATURE
   <222> (27)..(27)
   <223> Lys-NH(epsilon) octanoyl
<400> 37
<21 0> 38
   <211> 39
   <212> PRT
   <213> Artificial
<220>
   <223> Artificial Sequence
<220>
   <221> MISC_FEATURE
   <223> Description of Artificial Sequence: Exendin agonist peptide
<220>
   <221> MISC_FEATURE
   <223> c-term amidation
<220>
   <221> MISC_FEATURE
   <222> (1)..(1)
   <223> 4-imidazopropionyl
<220>
   <221> MISC_FEATURE
   <222> (28)..(28)
   <223> Lys-NH(epsilon) octanoyl
<400> 38
<210> 39
   <211> 39
   <212> PRT
   <213> Artificial
<220>
   <223> Artificial Sequence
<220>
   <221> MISC FEATURE
   <223> Description of Artificial Sequence: Exendin agonist peptide
<220>
   <221> MISC FEATURE
   <223> c-term amidation
<220>
   <221> MISC_FEATURE
   <222> (1)..(1)
   <223> 4-imidazopropionyl
<220>
   <221> MISC_FEATURE
   <222> (28)..(28)
   <223> Lys-NH(epsilon) octanoyl
<400> 39
<21 0> 40
   <211> 39
   <212> PRT
   <213> Artificial
<220>
   <223> Artificial Sequence
<220>
   <221> MISC_FEATURE
   <223> Description of Artificial Sequence: Exendin agonist formula peptide
<220>
   <221> MISC_FEATURE
   <223> c-term may be amidated
<220>
   <221> MISC_FEATURE
   <223> See specification as filed for detailed description of substitutions and preferred embodiments.
<220>
   <221> MISC_FEATURE
   <222> (1)..(1)
   <223> His, Arg, Tyr or 4-imidazopropionyl
<220>
   <221> MISC_FEATURE
   <222> (2)..(2)
   <223> Ser, Gly, Ala or Thr
<220>
   <221> MISC_FEATURE
   <222> (3)..(3)
   <223> Asp or Glu
<220>
   <221> MISC_FEATURE
   <222> (6)..(6)
   <223> Phe, Tyr or naphthylalanine
<220>
   <221> MISC_FEATURE
   <222> (7)..(7)
   <223> Thr or Ser
<220>
   <221> MISC_FEATURE
   <222> (8)..(8)
   <223> Ser or Thr
<220>
   <221> MISC_FEATURE
   <222> (9)..(9)
   <223> Asp or Glu
<220>
   <221> MISC_FEATURE
   <222> (10)..(10)
   <223> Leu, Ile, Val, pentylglycine or Met
<220>
   <221> MISC_FEATURE
   <222> (14)..(14)
   <223> Leu, Ile, Val, pentylglycine or Met
<220>
   <221> MISC_FEATURE
   <222> (22)..(22)
   <223> Phe, Tyr or naphthylalanine
<220>
   <221> MISC_FEATURE
   <222> (23)..(23)
   <223> Ile, Val, Leu, pentylglycine, tert-butylglycine or Met
<220>
   <221> MISC_FEATURE
   <222> (24)..(24)
   <223> Glu or Asp
<220>
   <221> MISC_FEATURE
   <222> (25)..(25)
   <223> Trp, Phe, Tyr, or naphthylalanine
<220>
   <221> MISC_FEATURE
   <222> (27)..(28)
   <223> Lys Asn, Asn Lys, Lys-NH(epsilon)-R Asn, or Asn Lys-NH(epsilon)-R, wherein R is Lys, Arg, C1-C10 straight chain or branched alkanoyl or cycloalkylalkanoyl
<220>
   <221> MISC_FEATURE
   <222> (31)..(31)
   <223> Pro, homoproline, 3Hyp, 4Hyp, thioproline, N-alkylglycine, N-alkylpentylglycine or N-alkylalanine
<220>
   <221> MISC_FEATURE
   <222> (36)..(38)
   <223> Pro, homoproline, 3Hyp, 4Hyp, thioproline, N-alkylglycine, N-alkylpentylglycine or N-alkylalanine
<220>
   <221> MISC_FEATURE
   <222> (39)..(39)
   <223> Ser, Thr or Tyr
<400> 40